# EUROPEAN PATENT APPLICATION

(11) **EP 4 847 494 A1**
(43) Date of publication of application: **02.12.2026**
(21) Application number: 25745460.3
(22) Date of filing: 15.01.2025
(51) Int. Cl.: C07D 307/52, C08G 59/50, C09D 163/00

(54) **AMINE COMPOUND, CURING AGENT COMPOSITION COMPRISING SAME, COATING COMPOSITION, AND PRODUCT**

(30) Priority: 23.01.2024 KR 20240010036
(71) Applicant: Kuk Do Chemical Co., Ltd., Seoul 08588 (KR)
(72) Inventor: PARK, Chan Ho, Seoul 08588 (KR); OK, Chang Yul, Seoul 08588 (KR); PARK, Min Jun, Seoul 08588 (KR); LEE, Ga Young, Seoul 08588 (KR); SHIN, Jae Uk, Seoul 08588 (KR); KIM, Jae Il, Seoul 08588 (KR); KIM, Dae-Kwon, Seoul 08588 (KR); NA, Sung- Hyuk, Seoul 08588 (KR)
(74) Representative: Vossius & Partner Patentanwälte Rechtsanwälte mbB
(86) International application number: PCT/KR2025/000920
(87) International publication number: WO 2025/159443

(57) **Abstract**

Disclosed is an amine compound that exhibits low irritation and low corrosivity due to low permeability into skin, and exhibits low viscosity properties when applied to a solvent-free curing agent composition. According to one aspect, an amine compound having an active hydrogen equivalent weight of 44 to 120, a curing agent composition including the same, a coating composition including the same, and a product manufactured using the coating composition are provided.

## Description

### Technical Field

The present disclosure relates to an amine compound having low viscosity and low skin irritation properties, a curing agent composition including the same, a coating composition, and a product including the same.

### Background Art

Epoxy resins are used in various applications such as various coatings, adhesives, paints and composite formulations due to their excellent adhesion and chemical resistance. The epoxy resin includes a bisphenol A-type epoxy resin, a bisphenol F-type epoxy resin, a polyfunctional epoxy resin, a novolac epoxy resin and the like, and the epoxy resin may be cured or crosslinked by an amine-based curing agent composition.

Solvent-types have been mainly used as an existing curing agent composition, however, due to environmental regulations on volatile organic compounds (VOCs), high-solid curing agent compositions, water-based curing agent compositions, solvent-free curing agent compositions and the like are being developed as various attempts to replace organic solvents or reduce the used amount thereof. However, these curing agent compositions have a problem in that it is difficult to control viscosity or pot life when used together with an epoxy resin. Accordingly, in a solvent-free curing agent composition, methods for controlling the viscosity of the curing agent composition by adjusting the content of free amine or using a low-molecular compound such as benzyl alcohol as a diluent have been recently proposed. However, the free amine and the low-molecular compound such as benzyl alcohol cause irritation and corrosivity to the skin of workers during painting work, requiring caution in handling.

### DISCLOSURE

### Technical Problem

One aspect of the present disclosure is directed to providing an amine compound having low irritation or corrosivity to skin, a curing agent composition having low viscosity including the same, a coating composition including the same, and a product including the same.

### Technical Solution

A first aspect of the present disclosure provides an amine compound having a structure of the following Chemical Formula 1, and having an active hydrogen equivalent weight (A.H.E.W.) of 44 to 120.

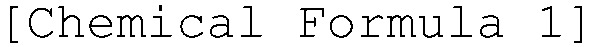

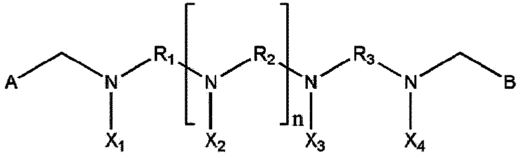

In Chemical Formula 1, R₁, R₂ and R₃ are each independently the same or different C₂-C₁₀ alkylene groups; A and B are each independently a substituted or unsubstituted C₆-C₁₄ aryl group; or a substituted or unsubstituted C₃-C₁₄ heteroaryl group, provided that at least one of A and B is a substituted or unsubstituted C₃-C₁₄ heteroaryl group, X₁, X₂, X₃ and X₄ are each independently A, B or a hydrogen atom, and n is 0, 1, 2, 3, 4, 5, 6 or 7.

According to a second aspect of the present disclosure, there may be provided a method for preparing an amine compound, the method comprising: preparing an amine compound by subjecting reactants including at least one polyalkyleneamine and at least one substituted or unsubstituted heteroaryl aldehyde to a hydrogenation reaction in the presence of a metal catalyst.

According to a third aspect of the present disclosure, in the first or second aspect, the amine compound may be a reaction product of the reactants including at least one polyalkyleneamine and at least one substituted or unsubstituted heteroaryl aldehyde.

According to a fourth aspect of the present disclosure, in the second or third aspect, the polyalkyleneamine may be a polyfunctional amine having three or more active amine hydrogens.

According to a fifth aspect of the present disclosure, in any one of the second to fourth aspects, the polyalkyleneamine may be at least one selected from the group consisting of diethylenetriamine, triethylenetetramine, N-3-aminopropyl ethylenediamine, N,N'-bis(3-aminopropyl)ethylenediamine, N,N,N'-tris(3-aminopropyl)ethylenediamine, N,N,N',N'-tetrakis(3-aminopropyl)ethylenediamine, N-3-aminopropyl-1,3-propanediamine, N,N'-bis(3-aminopropyl)-1,3-propanediamine, N,N,N'-tris(3-aminopropyl)-1,3-propanediamine, and N,N,N',N'-tetrakis(3-aminopropyl)-1,3-propanediamine.

According to a sixth aspect of the present disclosure, in any one of the second to fifth aspects, the polyalkyleneamine and the substituted or unsubstituted heteroaryl aldehyde may have a molar ratio of 1:1 to 1:2.5.

According to a seventh aspect of the present disclosure, in any one of the second to sixth aspects, the reactants may further include at least one substituted or unsubstituted aryl aldehyde.

According to an eighth aspect of the present disclosure, in any one of the second to seventh aspects, the molar ratio of the substituted or unsubstituted heteroaryl aldehyde may be greater than a molar ratio of the substituted or unsubstituted aryl aldehyde.

According to a ninth aspect of the present disclosure, in the eighth aspect, the substituted or unsubstituted heteroaryl aldehyde and the substituted or unsubstituted aryl aldehyde may have a molar ratio of 100:0 to 51:49.

According to a tenth aspect of the present disclosure, in any one of the first to ninth aspects, the heteroaryl group may include at least one selected from the group consisting of nitrogen, oxygen, phosphorus, sulfur and selenium.

According to an eleventh aspect of the present disclosure, in any one of the first to tenth aspects, when A or B has substituents, the substituents may be each independently at least one selected from the group consisting of -CH₂OH, -CH₂Y, -R₄CHO, -R₅, -CHO and -COOH, wherein Y may be a halogen atom, R₄ may be each independently a substituted or unsubstituted C₁-C₁₀ alkylene group, and R₅ may be a substituted or unsubstituted C₁-C₁₀ alkyl group. For example, A or B may include one or more substituents.

According to a twelfth aspect of the present disclosure, there is provided a curing agent composition comprising the amine compound according to any one of the first, and third to eleventh aspects.

According to a thirteenth aspect of the present disclosure, in any one of the second to twelfth aspects, the reactants include at least two polyalkyleneamines, and the amine compound may have a viscosity of 500 cps or less at 25°C.

According to a fourteenth aspect of the present disclosure, there is provided a coating composition comprising: the amine compound according to any one of the first, and third to thirteenth aspects; and an epoxy resin.

According to a fifteenth aspect of the present disclosure, there is provided a product comprising a cured material obtained by curing the coating composition according to the fourteenth aspect. As a non-limiting example, the product may be an adhesive, a coating, a primer, a sealant, a curing agent, a construction product, a flooring product, or a composite product thereof.

### Advantageous Effects

An amine compound according to one aspect of the present disclosure can be applied to a solvent-free curing agent composition. The solvent-free curing agent composition is safe due to its low irritation or corrosivity to skin, allows spray coating due to its low viscosity, and enables easy control of pot life.

### Brief Description of Drawings

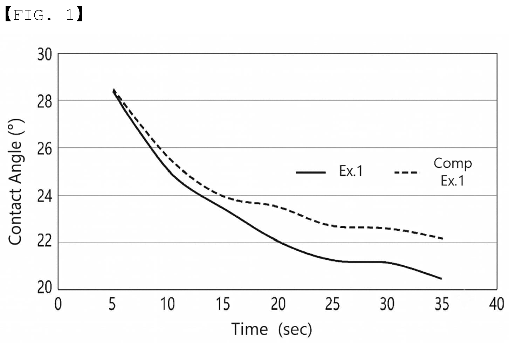

FIG. 1 is a graph showing changes in contact angles of droplets of synthesized products obtained in Example and Comparative Example of the present disclosure in accordance with ASTM D 5946.

### Mode for Invention

Hereinafter, each constitution of the present disclosure will be described in more detail so that those having common knowledge in the art may readily carry out the present disclosure, however, this is just one example, and the scope of a right of the present disclosure is not limited by the following.

The term "include (comprise)" used in the present specification is used to list materials, compositions, devices and methods useful for the present disclosure, and is not limited to the listed examples.

The terms "about" and "substantially" used in the present specification are used to refer to, considering unique manufacturing and material tolerances, a range of the number or degree or as a meaning close thereto, and are used to prevent infringers from unfairly using the disclosure stating precise or absolute numbers provided to help understand the present disclosure.

In the present specification, a numerical range indicated by using a term "to" represents a numerical range including values described before and after the term as a lower limit value and an upper limit value, respectively. For example, when "a to b" is described in the specification, it may be understood that a or more and b or less (a-b) is described.

In the present specification, when numerical values as upper and lower limits of any numerical range are each disclosed in multiple numbers, the numerical range disclosed in the present specification may be understood as any numerical range having any one of the multiple lower limit values and any one of the multiple upper limit values as a lower limit value and an upper limit value, respectively. For example, when a or more, or b or more; and c or less or d or less are described, it may be understood that a or more and c or less, a or more and d or less, b or more and c or less, or b or more and d or less are described.

In the present specification, unless otherwise defined below, the term "substituted" is defined such that at least one hydrogen atom is replaced by any one selected from the group consisting of a halogen atom, a hydroxyl group, a carboxyl group, a nitro group, an amine group, a sulfide group, a thiol group, an alkoxy group, an acetoxy group, a nitrile group, an aldehyde group, an ether group, an ester group, an acetal group, a ketone group, a C₁-C₃₀ alkyl group, a C₂-C₃₀ alkenyl group, a C₂-C₃₀ alkynyl group, a C₁-C₄₀ alkylsilyl group, a C₅-C₄₀ arylsilyl group, a C₃-C₃₀ cycloalkyl group, a C₃-C₃₀ allyl group, a C₆-C₃₀ aryl group, a heterocyclic group (for example: a C₂-C₃₀ heterocycloalkyl group, a C₃-C₃₀ heteroaryl group), derivatives thereof and combinations thereof. Herein, when being adjacent to each other, the respective substituents may bond to each other to form a substituted or unsubstituted fused ring or spiro structure.

"Skin irritation" and "skin corrosivity" used in the present specification may be evaluated by a Primary Irritation Index (P.I.I.) according to the skin irritation test method based on OECD guideline TG 404. When the P.I.I. is 5 or more, it may be evaluated as having strong skin irritation and corrosivity.

One embodiment of the present disclosure relates to an amine compound having a structure of the following Chemical Formula 1, wherein the amine compound has an active hydrogen equivalent weight (A.H.E.W.) of 44 to 120.

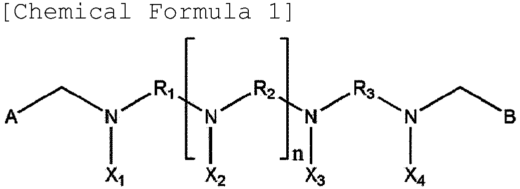

In Chemical Formula 1, R₁, R₂ and R₃ are each independently the same or different C₂-C₁₀ alkylene groups, A and B are each independently a substituted or unsubstituted C₆-C₁₄ aryl group; or a substituted or unsubstituted C₃-C₁₄ heteroaryl group, provided that at least one of A and B is a substituted or unsubstituted C₃-C₁₄ heteroaryl group, X₁, X₂, X₃ and X₄ are each independently A, B or a hydrogen atom, and n is 0, 1, 2, 3, 4, 5, 6 or 7.

The heteroaryl group may include at least one of heteroatoms selected from the group consisting of nitrogen, oxygen, phosphorus, sulfur and selenium, and may preferably include 1 to 4 heteroatoms.

When A or B has substituents, the substituents may be each independently at least one selected from the group consisting of -CH₂OH, -CH₂Y, -R₄CHO, R₅, CHO and COOH, wherein Y may be a halogen atom, R₄ may be each independently a substituted or unsubstituted C₁-C₁₀ alkylene group, and R₅ may be a substituted or unsubstituted C₁-C₁₀ alkyl group.

The active hydrogen equivalent weight (A.H.E.W.) of the amine compound may be calculated as a ratio of a molecular weight of the amine compound to the number of active hydrogens of amine included in the amine compound. Herein, the molecular weight of the amine compound may be measured using gel permeation chromatography (GPC), and the number of active hydrogens may be analyzed using ¹H-NMR (nuclear magnetic resonance).

In an existing solvent-free curing agent composition, curability and low viscosity properties of the composition are secured by increasing the content of free amine present in an unreacted state, and adjusting the active hydrogen equivalent weight to less than 44, however, skin irritation is high in the corresponding range.

It is confirmed that, when the active hydrogen equivalent weight of the amine compound of the present disclosure is adjusted to 44 to 120, curability suitable for coating and product formation is secured, and at the same time, skin irritation that is at least 50% lower than that of the existing one is exhibited. Specifically, the amine compound may have an active hydrogen equivalent weight of 44 or more, 45 or more, 50 or more, 55 or more, 60 or more, 65 or more, 70 or more, 75 or more, 80 or more, 85 or more, 90 or more, 95 or more, 100 or more, 105 or more or 115 or more, or 120 or less, 115 or less, 110 or less, 105 or less, 100 or less, 95 or less, 90 or less, 85 or less, 80 or less, 75 or less, 70 or less, 65 or less, 60 or less, 55 or less, 50 or less or 45 or less. Preferably, low skin irritation with a P.I.I. of 3.5 or less may be exhibited when the amine compound has an active hydrogen equivalent weight of 45 or more and 110 or less.

The amine compound may have a weight average molecular weight of 180 g/mol to 450 g/mol. Preferably, the amine compound may have a weight average molecular weight of 183.2 g/mol to 414.5 g/mol. Herein, by adjusting the weight average molecular weight of the amine compound in the above-mentioned numerical range, appropriate viscosity of the curing agent composition may be achieved, and safety for the human body may be ensured while providing chemical resistance, water resistance and low viscosity properties. For example, the weight average molecular weight of the amine compound may be measured by any one selected from the group consisting of gel permeation chromatography (GPC), mass spectrometry (MS) and a combination thereof. When the amine compound is analyzed by GPC, polystyrene (PS) may be selected as a standard sample, THF as a solvent, and KF-801/802/803 from Shodex as a column.

The amine compound may be a reaction product of reactants including at least one polyalkyleneamine and at least one substituted or unsubstituted heteroaryl aldehyde. The polyalkyleneamine and the substituted or unsubstituted heteroaryl aldehyde may react in the presence of a metal catalyst to form an imine, and may form an amine compound modified with a substituted or unsubstituted heteroaryl through a hydrogenation reaction. The amine compound is for use in a curing agent composition, and the curing agent composition may be used for curing or crosslinking an epoxy resin. Specifically, the curing agent composition may be a solvent-free type.

In an existing solvent-free curing agent composition, an amine compound in which polyalkyleneamine is substituted with substituted or unsubstituted aryl is used to improve chemical resistance and water resistance of a coating or product formed from a curing agent composition. However, the amine compound modified with a substituted or unsubstituted aryl has a structure similar to a surfactant in which a terminal including the aryl is lipophilic and a core including the amine is hydrophilic, thereby having high permeability into human skin to cause skin irritation or destroy a skin barrier, and the aryl structure is toxic to the human body, so that strong irritation and corrosivity to the skin (6.0 or more based on P.I.I.) may be exhibited.

The present disclosure provides an amine compound having a structure with reduced permeability, irritation and corrosivity to skin by modifying the amine compound with heteroaryl instead of the aryl, or modifying the amine compound with the aryl and heteroaryl, and a curing agent composition including the same. In other words, the amine compound may be modified with at least substituted or unsubstituted heteroaryl, and when used in the curing agent composition, it may provide safety for the human body while providing chemical resistance, water resistance and low viscosity properties. The amine compound modified only with heteroaryl according to one embodiment of the present disclosure exhibits weak skin irritation of less than 4 based on the P.I.I., and less than 3.5 in another embodiment. The amine compound modified with heteroaryl and the aryl according to another embodiment may exhibit skin irritation of less than 5 based on the P.I.I., and less than 4 according to another embodiment.

The polyalkyleneamine may be a polyfunctional amine having three or more active amine hydrogens. For example, the polyalkyleneamine may be at least one selected from the group consisting of diethylenetriamine, triethylenetetramine, N-3-aminopropyl ethylenediamine, N,N'-bis(3-aminopropyl)ethylenediamine, N,N,N'-tris(3-aminopropyl)ethylenediamine, N,N,N',N'-tetrakis(3-aminopropyl)ethylenediamine, N-3-aminopropyl-1,3-propanediamine, N,N'-bis(3-aminopropyl)-1,3-propanediamine, N,N,N'-tris(3-aminopropyl)-1,3-propanediamine and N,N,N',N'-tetrakis(3-aminopropyl)-1,3-propanediamine.

Preferably, a mixture of two or more polyfunctional amines may be used as the polyalkyleneamine. For example, the polyalkyleneamine may be a mixture of diethylenetriamine, triethylenetetramine and N,N'-bis(3-aminopropyl)ethylenediamine. A molar ratio of diethylenetriamine to triethylenetetramine in the reactants may be 2.0 times or more and 5.0 times or less. Specifically, the molar ratio of diethylenetriamine to triethylenetetramine may be 2.5 times or more, 3.0 times or more, 3.5 times or more or 4.0 times or more, or 4.5 times or less, 4.0 times or less, 3.5 times or less or 3.0 times or less, and preferably 2.5 times or more and 5.0 times or less. A molar ratio of N,N'-bis(3-aminopropyl)ethylenediamine to triethylenetetramine in the reactants may be 3.0 times or more and 7.0 times or less. Specifically, the molar ratio of N,N'-bis(3-aminopropyl)ethylenediamine to triethylenetetramine may be 3.5 times or more, 4.0 times or more, 4.5 times or more, 5.0 times or more, 5.5 times or more or 6.0 times or more, or 6.5 times or less, 6.0 times or less, 5.5 times or less, 5.0 times or less, 4.5 times or less, 4.0 times or less or 3.5 times or less, and preferably 3.5 times or more and 6.5 times or less.

The heteroaryl in the substituted or unsubstituted heteroaryl aldehyde may be C₃-C₁₄ heteroaryl, and the heteroaryl may include at least one selected from the group consisting of nitrogen, oxygen, phosphorus, sulfur and selenium. In the case of the nitrogen atom, a quaternized one may be included.

When the heteroaryl aldehyde has substituents, the substituents may be each independently at least one selected from the group consisting of -CH₂OH, -CH₂Y, -R₄CHO, -R₅, -CHO and -COOH, wherein Y may be a halogen atom, R₄ may be each independently a substituted or unsubstituted C₁-C₁₀ alkylene group, and R₅ may be a substituted or unsubstituted C₁-C₁₀ alkyl group.

Non-limiting examples of the heteroaryl may include monocyclic heteroaryl such as furyl, thiophenyl, pyrrolyl, imidazolyl, pyrazolyl, thiazolyl, isothiazolyl, isoxazolyl, oxazolyl, triazinyl, pyridyl, pyrazinyl, pyrimidinyl and pyridazinyl, polycyclic heteroaryl such as benzofuranyl, benzothiophenyl, isobenzofuranyl, benzimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl and benzodioxolyl, their corresponding N-oxides (for example, pyridyl N-oxide, quinolyl N-oxide), quaternary salts thereof, and the like. Preferably, the heteroaryl may be C₄-C₁₂ heteroaryl. More preferably, the heteroaryl may be furyl, and the heteroaryl aldehyde may be furfural. Furfural is a dehydration product of (pentose) sugars derived from various biomass sources such as oats, corn, wheat and sawdust, and an amine compound prepared from biomass such as furfural, and a curing agent composition including the same may be environmentally friendly through reducing carbon emissions.

The polyalkyleneamine and the substituted or unsubstituted heteroaryl aldehyde may have a reaction molar ratio of 1:1 to 1:2.5. Specifically, the reaction molar ratio may be 1:1.2 to 1:2.4, 1:1.5 to 1:2.4, or 1:8 to 1:2.2. Preferably, the reaction molar ratio may be 1:1.2 to 1:2.4. When the reaction molar ratio is less than 1:1, the amine compound exhibits an active hydrogen equivalent weight of less than 40, and a curing agent composition including the same may exhibit skin irritation. When the reaction molar ratio exceeds 1:2.5, a curing agent composition including the amine compound has increased viscosity, which may reduce workability as a solvent-free type.

The reactants may further include at least one substituted or unsubstituted aryl aldehyde. It will be understood that the reactants may include at least one polyalkyleneamine, at least one substituted or unsubstituted heteroaryl aldehyde and at least one substituted or unsubstituted aryl aldehyde, wherein at least a portion of the polyalkyleneamine is modified with substituted or unsubstituted heteroaryl.

In the substituted or unsubstituted aryl aldehyde, the aryl may be C₆-C₁₄ aryl, and may include a single or fused ring system. In addition, it may also include a structure in which one or more aryl groups are bonded through a chemical bond. Non-limiting examples of the aryl may include phenyl, naphthyl, biphenyl, anthryl, indenyl, fluorenyl, phenanthryl and the like. Preferably, the aryl may be C₆-C₁₀ aryl.

When the reactants include both the substituted or unsubstituted heteroaryl aldehyde and the substituted or unsubstituted aryl aldehyde, the molar ratio of the substituted or unsubstituted heteroaryl aldehyde may be greater than the molar ratio of the substituted or unsubstituted aryl aldehyde. Preferably, the substituted or unsubstituted heteroaryl aldehyde and the substituted or unsubstituted aryl aldehyde may have a molar ratio of 100:0 to 51:49. Specifically, the molar ratio may be 100:0 or more, 90:10 or more, 80:20 or more, 70:30 or more or 60:40 or more, and 90:10 or less, 80:20 or less, 70:30 or less, 60:40 or less or 51:49 or less. Preferably, the molar ratio may be 100:0 to 80:20. The amine compound satisfying the above-mentioned ratio may exhibit reduced skin irritation with a P.I.I. of less than 5, and the amine compound modified only with the heteroaryl may exhibit low skin irritation with a P.I.I. of less than 4.

Another embodiment of the present disclosure relates to a curing agent composition including the amine compound described above. Specifically, the curing agent composition may be a solvent-free type including the amine compound having the structure of Chemical Formula 1 and a curing accelerator.

The curing accelerator may be a tertiary amine. Non-limiting examples of the tertiary amine curing accelerator may include triethanolamine, diethylaminopropylamine, benzyldimethylamine, N,N'-dimethylpiperazine, N-methylpyrrolidine, N-methylhydroxypiperidine, N,N,N'N'-tetramethyldiaminoethane, N,N,N',N',N'-pentamethyldiethylenetriamine, tributylamine, trimethylamine, diethyldecylamine, triethylenediamine, N-methylmorpholine, N,N,N',N'-tetramethylpropane diamine, N-methyl piperidine, N,N'-dimethyl-1,3-(4-piperidino)propane, pyridine, 1,8-diazobicyclo[5.4.0]undec-7-ene, 1,8-diazabicyclo[2.2.2]octane, 4-dimethylaminopyridine, 4-(N-pyrrolidino)pyridine, triethylamine, 2,4,6-tris(dimethylaminomethyl)phenol, and the like.

The curing agent composition of the present disclosure may include an amine compound that exhibits low viscosity properties of 500 cps or less under a condition of 25°C even without a diluent such as benzyl alcohol. Preferably, the reactants may include two or more polyalkyleneamines and heteroaryl aldehyde, and the amine compound, which is a reaction product, may exhibit low viscosity properties of 300 cps or less under a condition of 25°C. The curing agent composition satisfying such low viscosity properties may be used in an environmentally friendly coating composition having a low solvent content.

The curing agent composition may include 100 parts by weight to 90 parts by weight of the amine compound having the structure of Chemical Formula 1, and 0 parts by weight to 10 parts by weight of the curing accelerator. When satisfying the above-described content range, the curing agent composition may exhibit compatibility with an epoxy resin and low viscosity properties, and may have excellent workability and curing properties.

Another embodiment of the present disclosure relates to a coating composition including the above-described amine compound and an epoxy resin, and a product manufactured using the coating composition. For example, the product may include a cured material obtained by curing the above-described curing agent composition and the above-described coating composition.

The epoxy resin may include at least one polyfunctional epoxy resin. The polyfunctional epoxy resin includes two or more epoxy groups per molecule, and may include glycidyl ethers of polyhydric phenols including glycidyl ethers of dihydric phenols. Non-limiting examples of the polyfunctional epoxy resin may include glycidyl ethers of resorcinol, hydroquinone, bis-(4-hydroxy-3,5-difluorophenyl)-methane, 1,1-bis-(4-hydroxyphenyl)-ethane, 2,2-bis-(4-hydroxy-3-methylphenyl)-propane, 2,2-bis-(4-hydroxy-3,5-dichlorophenyl)-propane, 2,2-bis-(4-hydroxyphenyl)-propane (commercially known as bisphenol A), bis-(4-hydroxyphenyl)-methane (commercially known as bisphenol F, which may include variable amount of 2-hydroxyphenyl isomer) and the like.

The coating composition may further include various additives depending on the manufacture or end-use of the product. The additives may include, for example, solvents (including water), accelerators, plasticizers, fillers, fibers such as glass or carbon fibers, pigments, pigment dispersants, rheology modifiers, thixotropes, flow or leveling aids, surfactants, defoamers, biocides, or any combination thereof.

The coating composition may be applied to painting or coating of heavy-duty metal objects or cement substrates, including ships, bridges, industrial plants and equipment, and floorings. The coating composition may be applied using a number of techniques using tools including sprays, brushes, rollers, paint mitts, and the like.

Non-limiting examples of the product may include coatings, adhesives, construction products, flooring products, or composite products. The coating may include various types and concentrations of pigments used in the fields of paints and primers. The coating composition may form a layer having a thickness in a range of 10 µm to 500 µm for use in protective coatings applied on a substrate. Non-limiting examples of the substrate may include concrete and various types of metals and alloys. For use in the flooring product or the construction product, the coating composition may form a layer having a thickness in a range of 50 µm to 10,000 µm depending on the type of the product and required final properties. A coating product imparting limited mechanical and chemical resistance may include a layer having a thickness in a range of 50 µm to 500 µm, whereas a coating product such as a leveling flooring material imparting high mechanical and chemical resistance may include a layer having a thickness in a range of 1,000 µm to 10,000 µm.

When the coating composition is applied to the construction product or the flooring product, the coating composition of the present disclosure may be used together with concrete or other materials commonly used in the construction industry. Non-limiting examples of the application of the coating composition include its use as a primer, a deep-penetrating primer, a coating, a curing agent, and/or a sealant for new or old concrete. As the primer or sealant, the coating composition of the present disclosure may be applied to a surface to improve adhesive bonding prior to coating application. When this relates to the fields of concrete and cement, the coating is a formulation used for application on a surface to create a protective or decorative layer or coating. Crack injection and crack filling products may also be manufactured from the composition disclosed herein. The coating composition of the present disclosure may be mixed with a cement material such as a concrete mixture to form polymer or modified cement, tile grout, or the like. Non-limiting examples of the product or composite product including the coating composition disclosed herein include tennis rackets, skis, bike frames, airplane wings, glass fiber-reinforced composites, and other molded products.

Hereinafter, the present disclosure will be described in more detail with reference to specific examples and experimental examples. The following examples and experimental examples are for illustrative purposes only, and the present disclosure is not limited by the following examples and experimental examples.

### Example 1

A Pd catalyst (3 g), DETA (31 g, 0.30 mol), TETA (16 g, 0.10 mol) and N,N'-bis(3-aminopropyl)ethylenediamine (N4) (87 g, 0.50 mol) were introduced to an autoclave batch reactor equipped with a stirrer at room temperature (25°C), followed by mixing for 30 minutes. After that, furfural (173 g, 1.80 mol) was added thereto over about 30 minutes, and the mixture was stirred at 100°C for 30 minutes, and then heated to 120°C. After reaching 120°C, hydrogen gas was supplied to maintain the pressure in a range of 50 bar to 60 bar, and the hydrogen gas consumption was checked. The reaction was terminated when the hourly hydrogen gas consumption reached 1 bar or less. After the reaction was completed, the reactor was cooled to 60°C, then the hydrogen gas was removed, and the Pd catalyst was removed using a filter. All moisture was removed from the residue under conditions of 120°C and 20 torr to obtain a synthesized product.

### Example 2

A synthesized product was obtained in the same manner as in Example 1, except that furfural (105 g, 1.10 mol) was added to DETA (31 g, 0.30 mol), TETA (16 g, 0.10 mol) and N4 (87 g, 0.50 mol).

### Example 3

A synthesized product was obtained in the same manner as in Example 1, except that furfural (210 g, 2.19 mol) was added to DETA (31 g, 0.30 mol), TETA (16 g, 0.10 mol) and N4 (87 g, 0.50 mol).

### Example 4

A synthesized product was obtained in the same manner as in Example 1, except that furfural (174 g, 1.81 mol) was added to DETA (34.4 g, 0.33 mol), TETA (11.2 g, 0.08 mol) and N4 (87 g, 0.50 mol).

### Example 5

A synthesized product was obtained in the same manner as in Example 1, except that furfural (174 g, 1.81 mol) was added to DETA (46.4 g, 0.45 mol), TETA (14 g, 0.10 mol) and N4 (61 g, 0.35 mol).

### Example 6

A synthesized product was obtained in the same manner as in Example 1, except that hydroxymethylfurfural (HMF) (227 g, 1.80 mol) was added to DETA (31 g, 0.30 mol), TETA (16 g, 0.10 mol) and N4 (87 g, 0.50 mol).

### Example 7

A synthesized product was obtained in the same manner as in Example 1, except that hydroxymethylfurfural (113.5 g, 0.90 mol) and furfural (86.5 g, 0.90 mol) were added to DETA (31 g, 0.30 mol), TETA (16 g, 0.10 mol) and N4 (87 g, 0.50 mol).

### Example 8

A synthesized product was obtained in the same manner as in Example 1, except that benzaldehyde (20 g, 0.19 mol) and furfural (156 g, 1.62 mol) were added to DETA (31 g, 0.30 mol), TETA (16 g, 0.10 mol) and N4 (87 g, 0.50 mol).

### Comparative Example 1

A synthesized product was obtained in the same manner as in Example 1, except that benzaldehyde (191 g, 1.80 mol) was added to DETA (31 g, 0.30 mol), TETA (16 g, 0.10 mol) and N4 (87 g, 0.50 mol).

### Comparative Example 2

A synthesized product was obtained in the same manner as in Example 1, except that furfural (86.5 g, 0.90 mol) was added to DETA (31 g, 0.30 mol), TETA (16 g, 0.10 mol) and N4 (87 g, 0.50 mol).

### Comparative Example 3

A synthesized product was obtained in the same manner as in Example 1, except that furfural (227 g, 2.36 mol) was added to DETA (31 g, 0.30 mol), TETA (16 g, 0.10 mol) and N4 (87 g, 0.50 mol).

### Reference Example 1

A synthesized product was obtained in the same manner as in Example 1, except that benzaldehyde (95.5 g, 0.90 mol) and furfural (86.5 g, 0.90 mol) were added to DETA (31 g, 0.30 mol), TETA (16 g, 0.10 mol) and N4 (87 g, 0.50 mol).

### Reference Example 2

A synthesized product was obtained in the same manner as in Example 1, except that salicylaldehyde (110 g, 0.90 mol) and furfural (86.5 g, 0.90 mol) were added to DETA (31 g, 0.30 mol), TETA (16 g, 0.10 mol) and N4 (87 g, 0.50 mol).

### Experimental Example 1. Determination of Properties for Synthesized Products of Examples and Comparative Examples

The compositions of the reactants used in the Examples and the Comparative Examples, and the properties (specifically, active hydrogen equivalent weight (A.H.E.W.) and viscosity) of the obtained synthesized products were determined, and the results are shown in Tables 1 and 2 below.

### Active Hydrogen Equivalent Weight

The active hydrogen equivalent weight was calculated according to the following Equation 1. 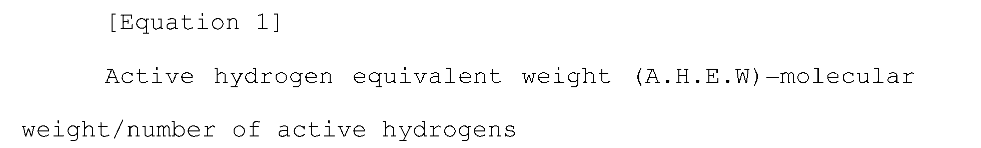[Equation 1] Active hydrogen equivalent weight (A.H.E.W)=molecular weight/number of active hydrogens

### Viscosity

The viscosity was measured at a temperature of 25°C using a Brookfield viscometer under torque conditions of 30% or more, with the marked portion at the pointed end of the spindle submerged in a Thermosel containing the sample.

**[Table 1]**

| | Example 1 | Example 2 | Example 3 | Example 4 | Example 5 | Example 6 | Example 7 | Example 8 |
|---|---|---|---|---|---|---|---|---|
| Furfural | 1.80 mol | 1.10 mol | 2.19 mol | 1.81 mol | 1.81 mol | - | 0.90 mol | 1.62 mol |
| Hydroxy methylfurfural | - | - | - | - | - | 1.80 mol | 0.90 mol | - |
| Benzald ehyde | - | - | - | - | - | - | - | 0.19 |
| Salicyl aldehyde | - | - | - | - | - | - | - | - |
| DETA | 0.30 mol | 0.30 mol | 0.30 mol | 0.33 mol | 0.45 mol | 0.30 mol | 0.30 mol | 0.30 mol |
| TETA | 0.10 mol | 0.10 mol | 0.10 mol | 0.08 mol | 0.10 mol | 0.10 mol | 0.10 mol | 0.10 mol |
| N4 | 0.50 mol | 0.50 mol | 0.50 mol | 0.50 mol | 0.35 mol | 0.50 mol | 0.50 mol | 0.50 mol |
| A.H.E.W. | 82.3 | 45.4 | 106.7 | 82.7 | 82.8 | 97.8 | 89.7 | 82.9 |
| Viscosity (@25°C) | 170 cps | 80 cps | 260 cps | 110 cps | 90 cps | 240 cps | 200 cps | 158 cps |

**[Table 2]**

| | Comparative Example 1 | Comparative Example 2 | Comparative Example 3 | Reference Example 1 | Reference Example 2 |
|---|---|---|---|---|---|
| Furfural | - | 0.90 mol | 2.36 mol | 0.90 mol | 0.90 mol |
| Hydroxymethylf urfural | - | - | - | - | - |
| Benzaldehyde | 1.80 mol | - | - | 0.90 mol | - |
| Salicylaldehyde | - | - | - | - | 0.90 mol |
| DETA | 0.30 mol | 0.30 mol | 0.30 mol | 0.30 mol | 0.30 mol |
| TETA | 0.10 mol | 0.10 mol | 0.10 mol | 0.10 mol | 0.10 mol |
| N4 | 0.50 mol | 0.50 mol | 0.50 mol | 0.50 mol | 0.50 mol |
| A.H.E.W. | 87 | 36.3 | 120.3 | 84.4 | 88.7 |
| Viscosity (@25°C) | 90 cps | 73 cps | 290 cps | 135 cps | 190 cps |

### Experimental Example 2. Determination of ASTM D 5946 Contact Angle for Synthesized Products of Example 1 and Comparative Example 1

The contact angles of the synthesized products obtained in Example 1 and Comparative Example 1 were determined according to ASTM D 5946. Specifically, a droplet of each synthesized product was dropped onto a solid surface, and the angle formed between the stationary droplet and the surface was measured. A smaller contact angle may be evaluated as indicating higher hydrophilicity and superior adhesion.

3 µL of each of the synthesized products of Example 1 and Comparative Example 1 was dropped onto a surface of a glass substrate using a quantitative dispenser, and then a change in the contact angle was measured starting from 1 second after the dropping. The contact angle was determined by measuring the angle calculated from height and length of a triangle formed by the height of the highest point of the droplet with reference to the substrate, and the length of the substrate formed when a perpendicular line is drawn from the highest point, and the change in the contact angle over time was measured for up to 35 seconds. The results are shown in FIG. 1.

Referring to FIG. 1, it was confirmed that, except immediately after the discharge of the droplet, the sample of Example 1 exhibited higher hydrophilicity than the sample of Comparative Example 1 over time. Such results are consistent with the expectation that the sample of Example 1, which includes an amine compound modified with heteroaryl, would exhibit improved hydrophilicity at the terminal end of the compound compared with the sample of Comparative Example 1, which includes an amine compound modified only with aryl.

### Experimental Example 3. TG 404 Test for Synthesized Products of Examples and Comparative Examples

A skin irritation evaluation test was performed on the synthesized products obtained in Examples 1, 6 and 8, and Comparative Examples 1 to 4 according to OECD guideline TG 404.

Specifically, the hair of rabbits (New Zealand White Rabbit, average weight 2.22 kg) was shaved, and using the midline of the scapular region of the shaved rabbits as an axis of symmetry, each site was employed to have a size of about 2.5 cm × 2.5 cm as an administration site. Among the sites, one site was designated as an untreated control site, and the other site was designated as a test substance administration site.

Using the synthesized products of the Examples and the Comparative Examples as undiluted test substances, 0.5 mL of each substance was uniformly applied onto a 2.5 cm × 2.5 cm patch. The patch was applied to the test substance administration site, while only a patch was applied to the untreated control site. The irritation and corrosivity tests were conducted by dividing them into an initial test and a confirmatory test. The initial test was performed by sequentially applying three patches, wherein the first patch was removed 3 minutes after administration, the second patch was removed 1 hour after administration, and the third patch was removed 4 hours after administration. Since no severe irritation and corrosivity were observed after the removal of each patch, the confirmatory test was performed using separate animals by applying one patch for 4 hours each. Skin reactions were evaluated at 1, 24, 48 and 72 hours after removing the patch, and the degree of skin irritation of the test substance was classified by calculating the Primary Skin Irritation Index (P.I.I.). After the application to each site, evaluation was performed according to Table 3 below, and the average scores thereof are summarized in Table 4. The P.I.I. according to the following Equation 2 was calculated using the scores, which is summarized in Table 4.

**[Table 3]**

| Erythema and Eschar Formation (hereinafter, "Erythema") | |
|---|---|
| No erythema | 0 |
| Very slight erythema (barely perceptible) | 1 |
| Well-defined erythema | 2 |
| Moderate to severe erythema | 3 |
| Severe erythema (beet-redness) and eschar formation | 4 |

| Edema Formation (hereinafter, "Edema") | |
|---|---|
| No edema | 0 |
| Very slight edema (barely perceptible) | 1 |
| Slight edema (edges of area well-defined by definite raising) | 2 |
| Moderate edema (raised approximately 1 mm) | 3 |
| Severe edema (raised more than 1 mm and extending beyond the area of exposure) | 4 |

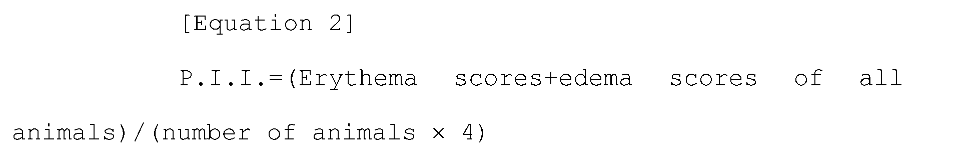

[Equation 2] P.I.I.=(Erythema scores+edema scores of all animals)/(number of animals × 4)

**[Table 4]**

| Sample | After 1 hour | | After 24 hours | | After 48 hours | | After 72 hours | | P.I.I. |
|---|---|---|---|---|---|---|---|---|---|
| | Eryth ema | Edema | Eryth ema | Edema | Eryth ema | Edema | Eryth ema | Edema | |
| Comparative Example 1 | 2 | 2 | 3 | 4 | 3 | 4 | 3 | 4 | 6.25 |
| Comparative Example 2 | 2 | 2 | 2 | 2 | 2 | 2 | 3 | 2 | 4.25 |
| Comparative Example 3 | 2 | 2 | 2 | 2 | 2 | 2 | 2 | 2 | 4.0 |
| Reference Example 1 | 2 | 0 | 3 | 2 | 3 | 3 | 3 | 3 | 4.75 |
| Reference Example 2 | 2 | 0 | 3 | 2 | 3 | 3 | 3 | 2 | 4.5 |
| Example 1 | 2 | 0 | 2 | 2 | 2 | 2 | 2 | 0 | 3.00 |
| Example 6 | 2 | 0 | 3 | 2 | 2 | 2 | 2 | 0 | 3.25 |
| Example 8 | 2 | 2 | 2 | 2 | 2 | 2 | 2 | 1 | 3.75 |

## Claims

1. An amine compound having a structure of the following Chemical Formula 1, and having an active hydrogen equivalent weight (A.H.E.W.) of 44 to 120: 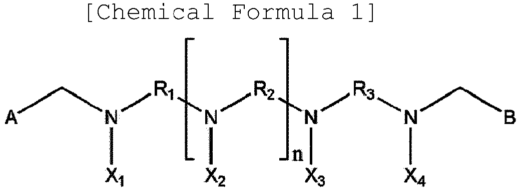
wherein, in Chemical Formula 1,
R₁, R₂ and R₃ are each independently the same or different C₂-C₁₀ alkylene groups;
A and B are each independently a substituted or unsubstituted C₆-C₁₄ aryl group; or a substituted or unsubstituted C₃-C₁₄ heteroaryl group, provided that at least one of A and B is a substituted or unsubstituted C₃-C₁₄ heteroaryl group;
X₁, X₂, X₃ and X₄ are each independently A, B or a hydrogen atom; and
n is 0, 1, 2, 3, 4, 5, 6 or 7.

2. The amine compound of claim 1, which is a reaction product of reactants including at least one polyalkyleneamine and at least one substituted or unsubstituted heteroaryl aldehyde.

3. The amine compound of claim 2, wherein the polyalkyleneamine is a polyfunctional amine having three or more active amine hydrogens.

4. The amine compound of claim 3, wherein the polyalkyleneamine is at least one selected from the group consisting of diethylenetriamine, triethylenetetramine, N-3-aminopropyl ethylenediamine, N,N'-bis(3-aminopropyl)ethylenediamine, N,N,N'-tris(3-aminopropyl)ethylenediamine, N,N,N',N'-tetrakis(3-aminopropyl)ethylenediamine, N-3-aminopropyl-1,3-propanediamine, N,N'-bis(3-aminopropyl)-1,3-propanediamine, N,N,N'-tris(3-aminopropyl)-1,3-propanediamine, and N,N,N',N'-tetrakis(3-aminopropyl)-1,3-propanediamine.

5. The amine compound of claim 2, wherein the polyalkyleneamine and the substituted or unsubstituted heteroaryl aldehyde have a molar ratio of 1:1 to 1:2.5.

6. The amine compound of claim 2, wherein the reactants further include at least one substituted or unsubstituted aryl aldehyde.

7. The amine compound of claim 6, wherein the molar ratio of the substituted or unsubstituted heteroaryl aldehyde is greater than a molar ratio of the substituted or unsubstituted aryl aldehyde.

8. The amine compound of claim 7, wherein the substituted or unsubstituted heteroaryl aldehyde and the substituted or unsubstituted aryl aldehyde have a molar ratio of 100:0 to 51:49.

9. The amine compound of claim 1, wherein the heteroaryl group includes at least one selected from the group consisting of nitrogen, oxygen, phosphorus, sulfur and selenium.

10. The amine compound of claim 1, wherein, when A or B has substituents, the substituents are each independently at least one selected from the group consisting of -CH₂OH, - CH₂Y, -R₄CHO, -R₅, -CHO and -COOH;
Y is a halogen atom;
R₄ is each independently a substituted or unsubstituted C₁-C₁₀ alkylene group; and
R₅ is a substituted or unsubstituted C₁-C₁₀ alkyl group.

11. A curing agent composition comprising the amine compound of claim 1.

12. The curing agent composition of claim 11, wherein the amine compound has a viscosity of 500 cps or less at 25°C.

13. A coating composition comprising:
the amine compound of claim 1; and
an epoxy resin.

14. A product comprising a cured material obtained by curing the coating composition of claim 13.
